(19) Europäisches Patentamt
European Patent Office
Office européen des brevets

(11) EP 0 952 817 B1

(12) FASCICULE DE BREVET EUROPEEN

(45) Date de publication et mention de la délivrance du brevet:
**04.07.2001 Bulletin 2001/27**

(21) Numéro de dépôt: **97953988.9**

(22) Date de dépôt: **31.12.1997**

(51) Int Cl.$^7$: **A61K 7/48**, A61K 7/06

(86) Numéro de dépôt international:
**PCT/FR97/02472**

(87) Numéro de publication internationale:
**WO 98/31334 (23.07.1998 Gazette 1998/29)**

# (54) COMPOSITION COSMETIQUE OU DERMATOLOGIQUE SOUS FORME D'UN GEL CONTENANT EN MELANGE UN COPOLYMERE ASSOCIATIF, UN TENSIOACTIF ET UN AGENT DE CONDITIONNEMENT INSOLUBLE

KOSMETISCHE UND DERMATOLOGISCHE ZUSAMMENSETZUNG IN GELFORM ENTHALTEND IN MISCHUNG EIN ASSOZIATIVES KOPOLYMER, EIN TENSID UND EIN KONDITIONIERUNGSMITTEL

COSMETIC OR DERMATOLOGICAL COMPOSITION IN THE FORM OF A GEL CONTAINING IN A MIXTURE AN ASSOCIATIVE COPOLYMER, A SURFACTANT AND A CONDITIONING AGENT

(84) Etats contractants désignés:
**DE ES FR GB IT**

(30) Priorité: **14.01.1997 FR 9700277**

(43) Date de publication de la demande:
**03.11.1999 Bulletin 1999/44**

(73) Titulaire: **L'OREAL**
**75008 Paris (FR)**

(72) Inventeurs:
- **DUPUIS, Christine**
**F-75018 Paris (FR)**
- **DUBIEF, Claude**
**F-78150 Le Chesnay (FR)**

(74) Mandataire: **Stalla-Bourdillon, Bernard**
**NONY & ASSOCIES**
**3, rue de Penthièvre**
**75008 Paris (FR)**

(56) Documents cités:
**EP-A- 0 452 758**
**EP-A- 0 507 693**
**US-A- 5 236 710**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 952 817 B1

Printed by Jouve, 75001 PARIS (FR)

## Description

La présente invention a pour objet une composition cosmétique ou dermatologique, à application topique, se présentant sous forme d'un gel aqueux contenant au moins un copolymère associatif, au moins un agent tensioactif de type non ionique, et au moins un agent de conditionnement insoluble choisi parmi les silicones, les hydrocarbures, les alcools gras et les esters gras.

Par l'expression "copolymère associatif", on entend selon l'invention, un copolymère amphiphile comportant à la fois des motifs hydrophiles et des motifs hydrophobes.

Il est déjà connu de réaliser des gels de forte viscosité à partir de copolymères associatifs avec une faible proportion d'un agent tensioactif.

Toutefois, on a constaté que ces gels s'ils constituaient de bons supports pour diverses applications cosmétiques ou dermatologiques, présentaient néanmoins une mauvaise texture rendant les gels difficilement préhensibles par les utilisateurs.

Après différentes études sur ces gels, on a constaté de façon surprenante et inattendue qu'il était possible d'en améliorer la texture et ainsi les rendre plus agréables et plus faciles à appliquer sur la peau, et plus particulièrement sur les cheveux, en y associant un certain pourcentage d'un agent de conditionnement insoluble choisi parmi les silicones, les hydrocarbures, les alcools gras et les esters gras.

On a en effet constaté que l'amélioration de la qualité des gels était nettement supérieure lorsque l'on utilisait un agent de conditionnement tel que défini ci-dessus, par rapport par exemple à une huile naturelle telle qu'une huile végétale.

Cette amélioration s'est par ailleurs avérée résulter du choix particulier de l'agent tensioactif utilisé. On a en effet constaté que l'emploi d'autres tensioactifs ne permettait pas de conduire à des résultats satisfaisants quant aux propriétés des gels obtenus.

La présente invention a donc pour objet une composition cosmétique ou dermatologique, à application topique, sous forme d'un gel aqueux, contenant :

(a) au moins un copolymère associatif choisi par copolymères non-réticulés, de type acrylique à chaîne hydrophobe, en une proportion de 0,8 à 20 % en poids par rapport au poids total de la composition,
(b) au moins un agent tensioactif du type non-ionique dans un rapport de 1/20 à 1/5 par rapport au copolymère associatif, mais présent en une proportion inférieure à 1 % en poids par rapport au poids total de la composition, et
(c) au moins un agent de conditionnement choisi parmi une silicone, un hydrocarbure, un alcool gras ou un ester gras, ledit agent de conditionnement étant présent en une proportion de 0,01 à 20 % en poids par rapport au poids total de la composition.

Les gels selon l'invention présentent un comportement viscoélastique. De préférence ils sont caractérisés par un angle de perte $\delta < 35$ et plus particulièrement $< 30$ dans la plage de fréquence $10^{-2}$ à 10 Hz et par une valeur du module complexe $G^* < 200\ N/m^2$ dans la plage de fréquence $10^{-2}$ à 10 Hz, de préférence par une valeur du module complexe $G^* > 100\ N/m^2$ à 10 Hz. Les mesures sont effectuées à 25°C à l'aide d'un rhéomètre à contrainte imposée (CARRIMED CSHR 100).

De préférence, la proportion en copolymère non-réticulé, du type acrylique à chaîne hydrophobe est comprise entre 1 et 10 % en poids par rapport au poids total de la composition.

Par l'expression "chaîne hydrophobe" on doit entendre selon l'invention, une chaîne alkyle ou alkényle, linéaire ou ramifiée, ayant de 8 à 32 atomes de carbone.

Parmi les copolymères non-réticulés, du type acrylique à chaîne hydrophobe, on peut citer en particulier, ceux choisis dans le groupe constitué par :

- les copolymères acide (méth)acrylique/acrylate d'éthyle/ acrylate d'alkyle $C_8$-$C_{22}$ tels que le produit "ACUSOL 823®" commercialisé par la Société ROHM & HAAS et le produit "IMPERON R®" par la Société HOECHST ;
- les copolymères acide acrylique/(méth)acrylate de lauryle tels que les produits "COATEX SX®" commercialisés par la Société COATEX ;
- les copolymères acide (méth)acrylique/acrylate d'alkyle en $C_1$-$C_{22}$/allyl éther d'alkyle $C_1$-$C_{22}$ polyéthoxylé dans lesquels au moins un des monomères contient une chaîne alkyle en $C_8$-$C_{22}$ tels que les produits "RHEOVIS-CR®", "RHEOVIS-$CR_2$®", "RHEOVIS-$CR_3$®" et "RHEOVIS-CRX®" commercialisés par la Société ALLIED COLLOIDS ;
- les terpolymères acide méthacrylique/acrylate d'éthyle/ acrylate de lauryle polyoxyéthyléné tels que le produit "RHEO 2000®" commercialisé par la Société COATEX ;
- les copolymères acide (méth)acrylique/acrylate d'éthyle/ méthacrylate de stéaryle polyoxyéthyléné tels que les produits "ACRYSOL 22®", "ACRYSOL 25®" et "DW-1206A®" commercialisés par la Société ROHM & HAAS ;
- les copolymères acide (méth)acrylique/acrylate d'éthyle/ acrylate de nonylphénol polyoxyéthyléné tels que le produit "RHEO 3000®" commercialisé par la Société COATEX ;

- les copolymères acide acrylique/monoitaconate de stéaryle ou de cétyle polyoxyéthyléné ou les copolymères acide acrylique/ monoitaconate de cétyle polyoxyéthyléné tels que les produits "8069-72A®" et "8069-72B®" commercialisés par la Société NATIONAL STARCH ;
- les copolymères acide (méth)acrylique/acrylate de butyle/ monomère hydrophobe comportant une chaîne grasse tels que le produit "8069-146A®" commercialisé par la Société NATIONAL STARCH ;
- les terpolymères acide acrylique/acrylate d'alkyle en $C_8$-$C_{20}$ (de préférence en $C_{19}$)acrylate de polyéthylèneglycol (de préférence de 20 à 30 moles d'oxyde d'éthylène) tels que le produit "DAPRAL GE 202®" commercialisé par la Société AKZO ;
- les copolymères acide (méth)acrylique/acrylate d'alkyle en $C_1$-$C_{22}$/monomère amphiphile comportant une chaîne hydrocarbonée en $C_8$-$C_{22}$ (par exemple alkyle ou alkényle) comprenant des groupements uréthannes tels que le produit "ADDITOL VXW 1312®" commercialisé par la Société HOECHST, et
- les polymères acryliques modifiés par des groupes hydrophobes à chaîne grasse (chaîne hydrocarbonée en $C_8$-$C_{22}$ tels que alkyle ou alkényle) tels que le produit "CS-0406®" commercialisé par la Société ROHM & HAAS.

Bien entendu, les copolymères décrits précédemment peuvent être utilisés seuls ou en mélange.

L'agent tensioactif selon l'invention du type non ionique des compositions selon l'invention est de préférence choisi parmi les alcools, les alphadiols, les alkylphénols ou les acides gras ceux-ci étant polyéthoxylés, polypropoxylés ou polyglycérolés et ayant une chaîne grasse comportant de 8 à 28 atomes de carbone,le nombre de groupes d'oxyde d'éthylène ou de propylène pouvant aller de 2 à 50 et celui de glycérol notamment de 2 à 30, les compolymères d'oxyde d'éthylène et de propylène, les condensats d'oxyde d'éthylène et de propoylène sur des alcools gras, les amines ou les amides gras polyéthoxylés ayant de préférence de 2 à 30 moles d'oxyde d'éthylène, les amides gras polyglycérolés comportant en moyenne 1 à 5 groupes glycérol,les diglycolamides polyglycérolés, les esters d'acides gras du sorbitan éventuellement oxyéthylénés, les esters d'acides gras du saccharose, les esters d'acides gras polyoxyalkylénés, les alkylpolyglycosides éventuellement oxyalkylénés, les esters d'alkylglucosides, les dérivés de N-alkylglucamine et de N-acylméthylglucamine, les aldobionamides et les oxydes d'amine.

Parmi les agents tensioactifs du type non-ionique particulièrement préférés, on peut citer notamment les esters de sorbitol et d'acides gras en $C_8$-$C_{22}$ éventuellement oxyéthyléné, les alkyl ($C_8$-$C_{22}$) polyglucosides tels que le produit commercialisé sous la dénomination d'"APG 300 GLYCOSIDE®" par la Société HENKEL.

L'agent tensio-actif de type non ionique peut éventuellement selon l'invention être associé à un agent tensioactif du type anionique ou amphotère.

Parmi les agents tensioactifs du type anionique, on peut citer notamment les sels, en particulier les sels alcalins et notamment de sodium, les sels d'ammonium, les sels d'amines, les sels d'aminoalcools ou les sels de magnésium des composés suivants : les alkylsulfates, les alkyléthersulfates, les alkylamidoéthersulfates, les monoglycérides sulfates, les alkylglycérylsulfonates, les alkylsulfonates, les alkyl-phosphates, les alkylamidesulfonates, les alkylarylsulfonates, les $\alpha$-oléfinesulfonates, les paraffines sulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acyltaurates, les N-acylaminoacides tels que les N-acylsarcosinates et les N-acylglutamates. On peut également citer comme agents tensioactifs anioniques pouvant être utilisés dans les compositions selon l'invention, les sels d'acides gras tels que les sels des acides undécénylique, oléique, ricinoléique, palmitique et stéarique, les acides d'huile de coprah ou d'huile de coprah hydrogénée et les acylhydroxyacides tels que les acyl-lactylates. On peut également utiliser des agents tensioactifs faiblement anioniques tels que les acides d'alkyl D-galactoside uroniques et leurs sels ainsi que les acides alkyléthers alkylamidoéthercarboxyliques polyoxyalkylénés ou leurs sels, le radical alkyle ou acyle de ces différents composés comportant de préférence de 8 à 22 atomes de carbone et les dérivés anioniques d'alkyle ($C_8$-$C_{22}$) polyglycosides (sulfate, sulfosuccinate, phosphate, iséthionate, éthercarboxylate, carbonate).

Parmi les agents tensioactifs du type amphotère on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant tel que par exemple, un groupe carboxylate, sulfonate, sulfate, phosphate ou phosphonate. On peut encore citer parmi les agents tensioactifs de type amphotère ou zwittérionique les sulfobétaines, les alkylamidoalkylbétaïnes, les alkylamidoalkylsulfobétaines, les dérivés d'imidazolium tels que ceux d'amphocarboxyglycinate ou d'amphocarboxypropionate.

Par l'expression "agent de conditionnement insoluble" on doit entendre selon l'invention une silicone, un hydrocarbure, un alcool gras ou un ester gras insoluble ou essentiellement insoluble dans l'eau (solubilité inférieure à 0,5 % en poids).

Lorsque l'agent de conditionnement de la composition selon l'invention est une silicone, celle-ci est généralement présente dans la composition selon l'invention, en une proportion de préférence comprise entre 0,05 à 5 % en poids par rapport au poids total de la composition.

Les silicones ou organopolysiloxanes utilisés dans la composition selon la présente invention, sont des huiles d'organopolysiloxanes ou des solutions organiques ou de gomme ou de résine d'organosiloxanes.
Parmi les organosiloxanes utilisés conformément à la préserte invention, on peut citer à titre non limitatif :

I. Les silicones volatiles

Celles-ci possèdent un point d'ébullition compris entre 60°C et 260°C. Parmi ce type de silicones, on cite :

(i) les silicones cycliques de 3 à 7 atomes de silicium et de préférence 4 à 5. Il s'agit, par exemple, de l'octaméthylcyclotétrasiloxane vendu sous le nom de "VOLATILE SILICONE 7207®" par la Société UNION CARBIDE ou "SILBIONE 70045 V2®" par la Société RHONE POULENC, le décaméthylcyclopentasiloxane vendu sous le nom de "VOLATILE SILICONE 7158®" par la Société UNION CARBIDE, "SILBIONE 70045 V5®" par la Société RHONE POULENC, ainsi que leurs mélanges.

On cite également les cyclopolymères du type diméthylsiloxane/méthylalkylsiloxane, tel que la "SILICONE VOLATILE FZ 3109®" vendue par la Société UNION CARBIDE, qui est un cyclocopolymère diméthylsiloxane/méthyloctylsiloxane ;

(ii) les silicones volatiles linéaires ayant 2 à 9 atomes de silicium et possédant une viscosité inférieure ou égale à $5.10^{-6}$ $m^2/s$ à 25°C. Il s'agit, par exemple, de l'hexaméthyldisiloxane vendu sous la dénomination "SILBIONE 70041 V0,65®" par la Société RHONE POULENC. Ce type de produit est décrit dans l'article de TODD & BYERS "Volatile silicone fluides for cosmetics", Cosmetics and Toiletries, Vol. 91, Jan 76, pages 27-32.

II. Les silicones non volatiles

Elles sont constituées principalement par les polyalkylsiloxanes, les polyarylsiloxanes, les polyalkylarylsiloxanes, les gommes et résines de silicone et les polysiloxanes organomodifiés, ainsi que leurs mélanges.
Parmi les polyalkylsiloxanes, on peut citer principalement les polydiméthylsiloxanes linéaires de viscosité supérieure à $5.10^{-6}$ $m^2/s$, et de préférence inférieure à 2,6 $m^2/s$ soit :

- à groupements terminaux triméthylsilyle, comme par exemple, et à titre non limitatif, les huiles "SILBIONE®" de la série 70047 commercialisées par la Société RHONE POULENC, l'huile "47 V 500.000®" de RHONE POULENC ou certaines "VISCASIL®" de la Société GENERAL ELECTRIC,
- à groupements terminaux trihydroxysilyle, tels que les huiles de la série "48 V®" de la Société RHONE POULENC.

Dans cette classe de polyalkylsiloxanes, on peut également mentionner les polyalkylsiloxanes vendus par la Société GOLDSCHMIDT sous les dénominations "ABILWAX 9800®" et "ABILWAX 9801®", qui sont des polyalkyl($C_1$-$C_{20}$)siloxanes.
Parmi les polyalkylarylsiloxanes, on peut citer les polydiméthylphénylsiloxanes, les polydiméthyldiphénylsiloxanes linéaires et/ou ramifiés, de viscosité $10^{-5}$ à $5.10^{-2}$ $m^2/s$ à 25°C, tels que par exemple :

- l'huile "RHODORSIL®" 763 de RHONE POULENC,
- les huiles "SILBIONE®" de la série 70641 de RHONE POULENC, telles que les huiles "SILBIONE 70641 V30®" et "SILBIONE 70641 V200®" de RHONE POULENC,
- le produit "DC 556®" Cosmetic Grad Fluid de DOW CORNING,
- les silicones de séries PK de BAYER, telles que la "PK20®",
- les silicones des séries PN, PH de BAYER, comme les "PN 1000®" et "PH 1000®",
- certaines huiles des séries SF de GENERAL ELECTRIC, telles que les "SF 1250®", "SF 1265®", "SF 1154®", "SF 1023®".

Les gommes de silicone, conformes à la présente invention, sont des polydiorganosiloxanes de forte masse moléculaire moyenne en nombre comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.
On cite, par exemple, les composés suivants :

- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],

- poly[(diméthylsiloxane)/(diphénylsiloxane)/ (méthylvinylsiloxane)].

On peut citer, par exemple, à titre non limitatif, les mélanges suivants :

1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401®" vendu par la Société DOW CORNING ;
2) les mélanges formés à partir d'une polydiméthylsiloxane avec une silicone cyclique, tel que le produit "SF 1214 SILICONE FLUID®" de GENERAL ELECTRIC, qui est une gomme "SE 30®" de PM 500.000 ($\overline{M}n$) solubilisée dans la "SF 1202 SILICONE FLUID®" (décaméthylcyclopentasiloxane) ;
3) les mélanges de deux PDMS de viscosités différentes, notamment d'une gomme PDMS et d'une huile PDMS, tels que les produits "SF 1236®" et "CF 1241®" de la Société GENERAL ELECTRIC. Le produit "SF 1236®" est le mélange d'une gomme "SE 30®" définie ci-dessus d'une viscosité de 20 $m^2/s$ et d'une huile "SF 96®" d'une viscosité de $5.10^{-6}$ $m^2/s$ (15 % de gomme "SE 30®" et 85 % d'huile "SF 96®").

Le produit "CF 1241®" est le mélange d'une gomme "SE 30®" (33 %) et d'une PDMS (67 %) de viscosité $10^{-3}$ $m^2/s$.

Les résines d'organopolysiloxanes utilisables conformément à l'invention, sont des systèmes siloxaniques réticulés renfermant les unités : $R_2SiO_{2/2}$, $RSiO_{3/2}$ et $SiO_{4/2}$ dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593®" ou ceux vendus sous les dénominations "SILICONE FLUID SS 4230" et "SILICONE FLUID SS 4267" par la Société GENERAL ELECTRIC et qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :

a) des groupements perfluorés tels que des trifluoroalkyles comme, par exemple, celles vendues par la Société GENERAL ELECTRIC sous les dénominations "FF.150 FLUOROSILICONE FLUID®" ou par la Société SHIN ETSU sous les dénominations "X-22-819®", "X-22-82®", "X-22-821®" et "X-22-822®" ;
b) des groupements hydroxyacylamino comme, par exemple, celles décrites dans la demande de brevet EP-A-0 342 834 et en particulier la silicone vendue par la Société Dow CORNING sous la dénomination "Q2-8413®" ;
c) des groupements thiols comme dans les silicones "X 2-8360®" de la Société Dow CORNING ou les "GP 72A®" et "GP 71®" de GENESEE ;
d) des groupements aminés substitués ou non, comme dans la "GP 4 SILICONE FLUID®" de GENESEE, la "GP 7100®" de GENESEE, la "Q2 8220®" de Dow CORNING, 1'"AFL 40®" d'UNION CARBIDE ou la silicone dénommée "Amodiméthicone" dans le dictionnaire CTFA ;
e) les groupements carboxylates, comme les produits décrits dans le brevet EP 186 507 de CHISSO CORPORATION ;
f) des groupements hydroxylés, comme les polyorganosiloxanes à fonction hydroxyalkyle, décrits dans la demande de brevet FR-85 16334, répondant à la formule suivante :

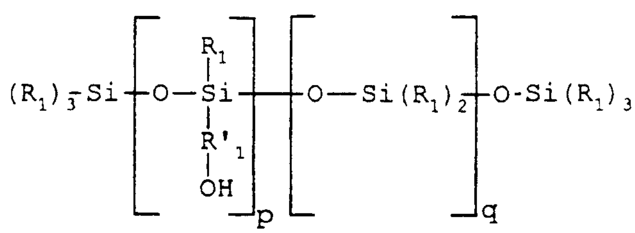

(I)

dans laquelle :

- les radicaux $R_1$, identiques ou différents, sont choisis parmi les radicaux méthyle et phényle, au moins 60 % en mole des radicaux $R_1$ étant méthyle ;

- le radical R'$_1$ est un chaînon alkylène divalent hydrocarboné en $C_2$-$C_{18}$ ;
- p est compris entre 1 et 30 inclus ;
- q est compris entre 1 et 150 inclus :

g) des groupements alcoxylés comme dans la Silicone copolymer "F 755®" de SWS SILICONES et les produits "ABILWAX 2428®", "ABILWAX 2434®", "ABILWAX 2440®" de la Société GOLDSCHMIDT ;
h) des groupements acyloxyalkyles, comme par exemple les polyorganopolysiloxanes décrits dans la demande de brevet FR-88 17433, répondant à la formule suivante :

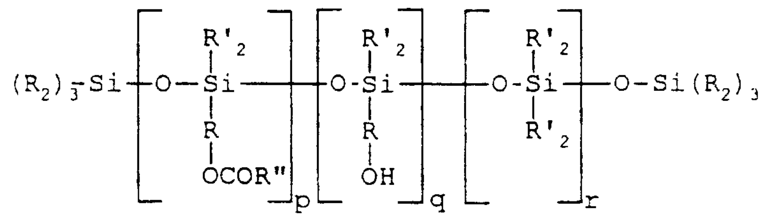 (II)

dans laquelle :

- $R_2$ désigne méthyle, phényle, OCOR", hydroxyle, un seul des $R_2$ par atome de silicium peut être OH ;
- R'$_2$ désigne méthyle, phényle, 60 % molaire au moins de l'ensemble des radicaux $R_2$ et R'$_2$ est méthyle ;
- R" désigne alcoyle ou alcényle en $C_8$-$C_{20}$ ;
- R désigne un alkylène hydrocarboné divalent, linéaire ou ramifié en $C_2$-$C_{18}$ ;
- r est compris entre 1 et 120 inclus ;
- p est compris entre 1 et 30 inclus ;
- q vaut 0 ou est inférieur à 0,5 p, p + q étant compris entre 1 et 30 inclus :

les polyorganosiloxanes de formule (II) peuvent contenir des groupements

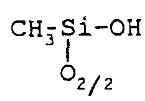

dans des proportions ne dépassant pas 15 % de la somme p + q + r ;
i) des groupements ammonium quaternaires, comme dans les produits "X2 81 08®" et "X2 81 09®", le produit "ABIL K 3270®" de la Société GOLDSCHMIDT ;
j) des groupements amphotères ou bétaïniques, tels que dans le produit vendu par la Société GOLDSCHMIDT sous la dénomination "ABIL B 9950®" ;
k) des groupements bisulfites, tels que dans les produits vendus par la Société GOLDSCHMIDT sous les dénominations "ABIL S 201®" et "ABIL S 255®" ;

Les polyorganosiloxanes particulièrement préférés, selon la présente invention, sont choisis parmi :

1) les silicones non volatiles du type polyalkylsiloxane linéaire à groupements terminaux triméthylsilyle, telles que les huiles "SILBIONE®" des séries 70047 et 47, telles que l'huile "47 V 500.000®", commercialisées par la Société RHONE POULENC ou du type polyalkylaryl-siloxane comme l'huile "SILBIONE 70641 V 200®" de la Société RHONE POULENC ;
2) les mélanges d'organosiloxanes et de silicones cycliques tels que la "Q2 1401®" de la Société Dow CORNING, la "SF 1214 SILICONE FLUID®" de la Société GENERAL ELECTRIC ;
3) les fluorosilicones de type polyalkylsiloxane à groupements terminaux triméthylsilyle et substituées sur la chaîne par des groupements trifluoropropyle telles que la fluorosilicone vendue par la Société SHIN ETSU sous la dénomination "X-22-821®".

Lorsque l'agent de conditionnement de la composition selon l'invention est un hydrocarbure, celui-ci peut être

un hydrocarbure, linéaire ou ramifié en $C_8$-$C_{300}$. Parmi les hydrocarbures liquides à température ambiante répondant à cette définition, on peut notamment citer l'isododécane, l'isohexadécane et ses isomères (tels que le 2,2,4,4,6,6-heptaméthylnonane), l'isoeicosane, l'isotétracosane, et les isomères desdits composés. On utilise de préférence selon l'invention l'isododécane ou l'un de ses isomères.

Lorsque l'agent de conditionnement est un alcool gras, celui-ci est du type linéaire ou ramifié, saturé ou insaturé, en $C_8$-$C_{22}$ et parmi ceux-ci on peut citer le butyl-2 octanol, l'alcool laurique, l'alcool oléique, l'alcool isocétylique et l'alcool isostéarique.

Lorsque l'agent de conditionnement est un ester gras, celui-ci peut être soit un ester d'un acide gras en $C_8$-$C_{22}$ et d'alcool en $C_1$-$C_{22}$ soit un ester d'un acide ou diacide en $C_1$-$C_7$ et d'un alcool gras en $C_8$-$C_{22}$. Parmi ces esters on peut citer le palmitate d'éthyle, d'isopropyle, d'éthyl-2-hexyle et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

Les hydrocarbures, les alcools gras ou les esters gras et leurs mélanges sont, tout comme les silicones, de préférence présents en une proportion comprise entre 0,05 à 5 % en poids par rapport au poids total de la composition.

Dans les compositions sous forme de gel aqueux selon l'invention, on peut introduire diverses substances actives présentant un intérêt cosmétique ou dermo-pharmaceutique.

Parmi ces substances actives on peut citer à titre d'exemple :

- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens ou les antibiotiques, les antiparasitaires, les antifongiques, les agents antiviraux, les agents anti-inflammatoires stéroïdiens ou les agents anti-inflammatoires non-stéroïdiens, des substances telles que les antagonistes de substance P, de CGRP ou de bradykinine ou les inhibiteurs de NO synthase, les agents anesthésiques, les agents antiprurigineux.

Comme autres substances actives on peut citer :

- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, les antiséborrhéiques, les antiacnéiques, les dérivés de pyrimidine, comme le 2,4-diamino 6-piperidinopyrimidine 3-oxyde ou "MINOXIDIL" ou encore ses nombreux dérivés, les agents favorisant la repousse des cheveux comme ceux décrits dans la demande de brevet EP 0648488, les agents antagonistes du calcium, des hormones ou des agents antiandrogènes.

Les compositions selon l'invention peuvent également contenir différents adjuvants utilisés en particulier en cosmétique tels que des parfums, des conservateurs, des filtres solaires, des séquestrants, des colorants, des agents acidifiants ou alcalinisants, des agents hydratants ou émollients, des agents réducteurs, des agents oxydants, des agents de conditionnement du cheveu ou de la peau non huileux, ainsi que d'autres adjuvants selon l'usage envisagé.

On va maintenant donner à titre d'illustration, plusieurs exemples de composition selon l'invention.

EXEMPLES

EXEMPLE 1 : *Gel de soins non rincé*

On prépare un gel non rincé par mélange des ingrédients suivants :

- Terpolymère acide acrylique/acrylate d'alkyle en $C_1$-$C_{18}$/méthacrylate de stéaryle polyoxyéthyléné à 20 moles d'oxyde d'éthylène commercialisé sous la dénomination de "ACRYSOL ICS-1®" par la Société ROHM & HAAS 1,0 g
- Ester laurique de sorbitol oxyéthyléné à 20 moles d'oxyde d'éthylène (Tween 20) 0,1 g
- Butyl-2 octanol (Isofol 12) 2,0 g
- Amino-2-méthyl-2-propanol-1 qs pH 7,5
- Eau qsp 100,0 g

Le gel obtenu présente une excellente texture et est particulièrement facile à appliquer sur la chevelure.

EXEMPLE 2 : *Gel de soins non rincé*

- Terpolymère acide acrylique/acrylate d'alkyle en C1-C18/méthacrylate de stéaryle polyoxyéthyléné à 20 moles d'oxyde d'éthylène commercialisé sous la dénomination de "ACRYSOL ICS-1®" par la Société Rohm & Hass 2,0 g
- Décylpolyglucose commercialisé sous la dénomination d'"APG 300 GLYCOSIDE®" par la Société Henkel 0,2 g
- α-ω-di OH polydiméthylsiloxane en solution à 14 % dans le mélange cyclotétra/cyclopentadiméthyl siloxane ("Q2-1401®" de Dow Corning) 20,0 g
- Amino-2-méthyl-2-propanol-1 qs pH 7,5
- Eau qsp 100,0 g

EXEMPLE 3 : *Gel de soins non rincé*

- Terpolymère acide (méth)acrylique/acrylate d'alkyle en $C_8$-$C_{22}$/allyl éther d'alkyle $C_1$-$C_{22}$ polyoxyéthyléné commercialisé sous la dénomination de "RHEOVIS-CR®" par la Société ALLIED COLLOIDS 4,0 g
- Décylpolyglucose commercialisé sous la dénomination de "APG 300 GLYCOSIDE®" par la Société HENKEL 0,2 g
- Polydiméthyl siloxane de viscosité 500 cst commercialisé sous la dénomination de "MIRASIL DM 500®" par la Société RHONE POULENC 3,0 g
- Amino-2-méthyl-2-propanol-1 qs pH 7,5
- Eau qsp 100,0 g

EXEMPLE 4 : *Gel de soins non rincé*

- Acide méthacrylique/acrylate d'éthyle/acrylate de nonylphénol polyoxyéthyléné commercialisé sous la dénomination de "RHEO 3000®" par la Société COATEX 2,0 g
- Ester laurique de sorbitol oxyéthyléné à 20 moles d'oxyde d'éthylène (Tween 20) 0,4 g
- Isohexadécane 2,0 g
- Amino-2-méthyl-2-propanol-1 qs pH 7,5
- Eau qsp 100,0 g

## Revendications

1. Composition cosmétique ou dermatologique à application topique, sous forme d'un gel aqueux, caractérisée par le fait qu'elle contient :

   (a) au moins un copolymère associatif choisi parmi les copolymères non-réticulés, de type acrylique à chaîne hydrophobe, en une proportion de 0,8 à 20 % en poids par rapport au poids total de la composition,
   (b) au moins un agent tensioactif du type non ionique dans un rapport de 1/20 à 1/5 par rapport au copolymère associatif, mais présent en une proportion inférieure à 1 % en poids par rapport au poids total de la composition, et
   (c) au moins un agent de conditionnement insoluble choisi parmi une silicone, un hydrocarbure, un alcool gras ou un ester gras, ledit agent de conditionnement étant présent en une proportion de 0,01 à 20 % en poids par rapport au poids total de la composition.

2. Composition selon la revendication 1, caractérisée par le fait que la proportion en copolymère non-réticulé, du type acrylique à chaîne hydrophobe est comprise entre 1 et 10 % en poids par rapport au poids total de la composition.

3. Composition selon l'une quelconque des revendications précédentes, caractérisée par le fait que ledit copolymère

non-réticulé, de type acrylique à chaîne hydrophobe est choisi dans le groupe constitué par :

- les copolymères acide (méth)acrylique/acrylate d'éthyle/ acrylate d'alkyle $C_8$-$C_{22}$ ;
- les copolymères acide acrylique/(méth)acrylate de lauryle ;
- les copolymères acide (méth)acrylique/acrylate d'alkyle en $C_1$-$C_{22}$/allyl éther d'alkyle $C_1$-$C_{22}$ polyéthoxylé dans lesquels au moins un des monomères contient une chaîne alkyle en $C_8$-$C_{22}$ ;
- les terpolymères acide méthacrylique/acrylate d'éthyle/ acrylate de lauryle polyoxyéthyléné ;
- les copolymères acide méthacrylique/acrylate d'éthyle/ méthacrylate de stéaryle polyoxyéthyléné ;
- les copolymères acide (méth)acrylique/acrylate d'éthyle/ acrylate de nonylphénol polyoxyéthyléné ;
- les copolymères acide acrylique/monoitaconate de stéaryle ou de cétyle polyoxyéthyléné ;
- les copolymères acide (méth)acrylique/acrylate de butyle/monomère hydrophobe comportant une chaîne grasse ;
- les terpolymères acide acrylique/acrylate d'alkyle en $C_8$-$C_{20}$/acrylate de polyéthylèneglycol ;
- les copolymères acide (méth)acrylique/acrylate d'alkyle en $C_1$-$C_{22}$/monomère amphiphile comportant une chaîne hydrocarbonée en $C_8$-$C_{22}$ ; et
- les polymères acryliques modifiés par des groupes hydrophobes à chaîne grasse.

**4.** Composition selon l'une quelconque des revendications précédentes, caractérisée par le fait que ledit agent tensioactif du type non-ionique est choisi parmi les esters de sorbitol et d'acides gras en $C_8$-$C_{22}$ éventuellement oxyéthylénés et les alkylpolyglucosides.

**5.** Composition selon l'une quelconque des revendications précédentes caractérisée par le fait qu'elle contient en outre au moins un agent tensioactif anionique et/ou amphotère.

**6.** Composition selon l'une quelconque des revendications précédentes caractérisée par le fait que la silicone est du type volatile ayant un point d'ébullition compris entre 60°C et 260°C.

**7.** Composition selon l'une quelconque des revendications 1 à 5, caractérisée par le fait que la silicone est du type non volatile et est choisie parmi les polyalkylsiloxanes, les polyarylsiloxanes, les polyalkylarylsiloxanes, les gommes et résines de silicone et les polysiloxanes organo-modifiés et leurs mélanges.

**8.** Composition selon l'une quelconque des revendications 1 à 5, caractérisée par le fait que l'hydrocarbure est linéaire ou ramifié, cyclique ou acyclique, en $C_8$-$C_{300}$.

**9.** Composition selon la revendication 8, caractérisée par le fait que l'hydrocarbure est choisi parmi l'isododécane, l'isohexadécane et ses isomères, l'isoeicosane, l'isotétracosane et leurs isomères.

**10.** Composition selon l'une quelconque des revendications 1 à 5, caractérisée par le fait que l'alcool gras est linéaire ou ramifié, saturé ou insaturé en $C_8$-$C_{22}$.

**11.** Composition selon la revendication 10, caractérisée par le fait que l'alcool gras est choisi parmi le butyl-2 octanol, l'alcool laurique, l'alcool oléique, l'alcool isocétylique, et l'alcool isostéarique.

**12.** Composition selon l'une quelconque des revendications 1 à 5, caractérisée par le fait que l'ester gras est un ester d'un acide gras en $C_8$-$C_{22}$ et d'un alcool en $C_1$-$C_{22}$ ou un ester d'un acide ou diacide en $C_1$-$C_7$ et d'un alcool gras en $C_8$-$C_{22}$.

**13.** Composition selon la revendication 12, caractérisée par le fait que l'ester gras est choisi parmi le palmitate d'éthyle, d'isopropyle, d'éthyl-2-hexyle, et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

**14.** Composition selon l'une quelconque des revendications précédentes, caractérisée par le fait qu'elle contient en outre au moins un adjuvant choisi parmi les parfums, les conservateurs, les filtres solaires, les séquestrants, les agents hydratants ou émollients, les agents réducteurs, les agents oxydants, les agents de conditionnement du cheveu ou de la peau non huileux, les colorants et les agents acidifiants ou alcalinisants.

## Patentansprüche

1. Kosmetische oder dermatologische Zubereitung zur topischen Anwendung in Form eines wässrigen Gels, dadurch gekennzeichnet, dass diese enthält:

   (a) mindestens ein Assoziationscopolymer, ausgewählt unter den nicht vernetzten Copolymeren vom Acryltyp mit hydrophober Kette, in einem Anteil von 0,8 bis 20 Gew.-%, bezogen auf das Gesamtgewicht der Zubereitung,
   (b) mindestens ein grenzflächenaktives Mittel nicht-ionischer Art in einem Verhältnis von 1/20 bis 1/5 gegenüber dem Assoziationscopolymer, das aber in einem Anteil von weniger als 1 Gew.-%, bezogen auf das Gesamtgewicht der Zubereitung, vorliegt, und
   (c) mindestens ein unlösliches Konditionierungsmittel, ausgewählt unter einem Silikon, einem Kohlenwasserstoff, einem Fettalkohol oder einem Fettester, wobei das Konditionierungsmittel in einem Anteil von 0,01 bis 20 Gew.-%, bezogen auf das Gesamtgewicht der Zubereitung, vorliegt.

2. Zubereitung nach Anspruch 1, dadurch gekennzeichnet, dass der Anteil des nicht vernetzten Copolymers vom Acryltyp mit hydrophober Kette 1 bis 10 Gew.-% beträgt, bezogen auf das Gesamtgewicht der Zubereitung.

3. Zubereitung nach einem der vorstehenden Ansprüche, dadurch gekennzeichnet, dass das nicht vernetzte Copolymer vom Acryltyp mit hydrophober Kette ausgewählt wird aus der Gruppe bestehend aus:

   - den Copolyeren von (Meth) acrylsäure/Ethylacrylat/$C_8$-$C_{22}$-Alkylacrylat;
   - den Copolymeren von Acrylsäure/Lauryl(meth)acrylat;
   - den Copolymeren von (Meth)acrylsäure/$C_1$-$C_{22}$-Alkylacrylat/polyethoxyliertem $C_1$-$C_{22}$-Alkylallylether, in denen mindestens eines der Monomere eine $C_8$-$C_{22}$-Alkylkette enthält;
   - den Terpolymeren von Methacrylsäure/Ethylacrylat/polyethoxyliertem Laurylacrylat;
   - den Copolymeren von Methacrylsäure/Ethylacrylat/polyethoxyliertem Stearylmethacrylat;
   - den Copolymeren von (Meth)acrylsäure/Ethylacrylat/polyethoxyliertem Nonylphenolacrylat;
   - den Copolymeren von Acrylsäure/polyethoxyliertem Stearyloder Cetylmonoitaconat;
   - den Copolyeren von (Meth)acrylsäure/Butylacrylat/hydrophobem Monomer mit Fettsäurekette;
   - den Terpolymeren von Acrylsäure/$C_8$-$C_{20}$-Alkylacrylat/Polyethylenglykolacrylat;
   - den Copolymeren von (Meth)acrylsäure/$C_1$-$C_{22}$-Alkylacrylat/ amphiphilem Monomer mit $C_8$-$C_{22}$-Kohlenwasserstoffkette; und
   - den Acrylpolymeren, die mit hydrophoben Gruppen einer Fettsäurekette modifiziert sind.

4. Zubereitung nach einem der vorstehenden Ansprüche, dadurch gekennzeichnet, dass das grenzflächenaktive Mittel nicht-ionischer Art ausgewählt wird unter den gegebenenfalls ethoxylierten $C_8$-$C_{22}$-Sorbitol- und -Fettsäureestern und den Alkylpolyglucosiden.

5. Zubereitung nach einem der vorstehenden Ansprüche, dadurch gekennzeichnet, dass diese außerdem mindestens ein anionisches und/oder amphoteres grenzflächenaktives Mittel enthält.

6. Zubereitung nach einem der vorstehenden Ansprüche, dadurch gekennzeichnet, dass das Silikon flüchtiger Art ist mit einem Siedepunkt zwischen 60 °C und 260 °C.

7. Zubereitung nach einem der Ansprüche 1 bis 5, dadurch gekennzeichnet, dass das Silikon nicht-flüchtiger Art ist und ausgewählt wird unter den Polyalkylsiloxanen, Polyarylsiloxanen, Polyalkylarylsiloxanen, den Silikongummis und -harzen und den organisch modifizierten Polysiloxanen, sowie Gemischen davon.

8. Zubereitung nach einem der Ansprüche 1 bis 5, dadurch gekennzeichnet, dass der Kohlenwasserstoff linear oder verzweigt, cyclisch oder nicht-cyclisch und $C_8$-$C_{300}$ ist.

9. Zubereitung nach Anspruch 8, dadurch gekennzeichnet, dass der Kohlenwasserstoff ausgewählt wird unter Isododecan, Isohexadecan und dessen Isomeren, Isoeicosan, Isotetracosan, und Isomeren davon.

10. Zubereitung nach einem der Ansprüche 1 bis 5, dadurch gekennzeichnet, dass der Fettalkohol linear oder verzweigt, gesättigt oder ungesättigt und $C_8$-$C_{22}$ ist.

**11.** Zubereitung nach Anspruch 10, dadurch gekennzeichnet, dass der Fettalkohol ausgewählt wird aus 2-Butyloctanol, Laurylalkohol, Oleylalkohol, Isocetylalkohol und Isostearylalkohol.

**12.** Zubereitung nach einem der Ansprüche 1 bis 5, dadurch ge kennzeichnet, dass der Fettester ein Ester einer $C_8$-$C_{22}$-Fettsäure und eines $C_1$-$C_{22}$-Alkohols und ein Ester einer $C_1$-$C_7$-Säure oder einer zweiwertigen $C_1$-$C_7$-Säure oder eines $C_8$-$C_{22}$-Fettalkohols ist.

**13.** Zubereitung nach Anspruch 12, dadurch gekennzeichnet, dass der Fettester ausgewählt wird aus Ethylpalmitat, Isopropylpalmitat, 2-Ethylhexylpalmitat, 2-Octyldecylpalmitat, Isopropylmyristat, Butylmyristat, Cetylmyristat, 2-Octyldecylmyristat, Butylstearat, Hexylstearat, Hexyllaurat, 2-Hexyldecyllaurat, Isononylisononanoat und Dioctylmalat.

**14.** Zubereitung nach einem der vorstehenden Ansprüche, dadurch gekennzeichnet, dass diese außerdem mindestens ein Hilfsmittel enthält, ausgewählt aus den Parfümen, Konservierungsmitteln, Sonnenschutzmitteln, Sequestriermitteln, Hydratisierungsmitteln oder Weichmachern, Reduktionsmitteln, Oxidationsmitteln, Konditionierungsmitteln für nicht-ölhaltige(s) Haar oder Haut, den farbgebenden Mitteln und den sauer oder basisch wirkenden Mitteln.

## Claims

**1.** Cosmetic or dermatological composition for topical application in the form of an aqueous gel, characterized in that it comprises:

(a) at least one associative copolymer chosen from non-crosslinked copolymers of type acrylic with a hydrophobic chain, in a proportion of 0.8 to 20% by weight with respect to the total weight of the composition,
(b) at least one surface-active agent of the nonionic type, in a ratio of 1/20 to 1/5 with respect to the associative copolymer but present in a proportion of less than 1% by weight with respect to the total weight of the composition, and
(c) at least one insoluble conditioning agent chosen from a silicone, a hydrocarbon, a fatty alcohol or a fatty ester, the said conditioning agent being present in a proportion of 0.01 to 20% by weight with respect to the total weight of the composition.

**2.** Composition according to Claim 1, characterized in that the proportion of non-crosslinked copolymer of the type acrylic with a hydrophobic chain is between 1 and 10% by weight with respect to the total weight of the composition.

**3.** Composition according to either one of the preceding claims, characterized in that the said non-crosslinked copolymer of type acrylic with a hydrophobic chain is chosen from the group composed of:

- (meth)acrylic acid/ethyl acrylate/$C_8$-$C_{22}$ alkyl acrylate copolymers;
- acrylic acid/lauryl (meth)acrylate copolymers;
- (meth)acrylic acid/$C_1$-$C_{22}$ alkyl acrylate/ polyethoxylated $C_1$-$C_{22}$ alkyl allyl ether copolymers, in which copolymers at least one of the monomers comprises a $C_8$-$C_{22}$ alkyl chain;
- methacrylic acid/ethyl acrylate/polyoxyethylenated lauryl acrylate terpolymers;
- methacrylic acid/ethyl acrylate/polyoxyethylenated stearyl methacrylate copolymers;
- (meth)acrylic acid/ethyl acrylate/polyoxyethylenated nonylphenol acrylate copolymers;
- acrylic acid/polyoxyethylenated stearyl or cetyl monoitaconate copolymers;
- (meth)acrylic acid/butyl acrylate/hydrophobic monomer comprising a fatty chain copolymers;
- acrylic acid/$C_8$-$C_{20}$ alkyl acrylate/polyethylene glycol acrylate terpolymers;
- (meth)acrylic acid/$C_1$-$C_{22}$ alkyl acrylate/ amphiphilic monomer comprising a $C_8$-$C_{22}$ hydrocarboncomprising chain copolymers; and
- acrylic polymers modified by hydrophobic groups with a fatty chain.

**4.** Composition according to any one of the preceding claims, characterized in that the said surface-active agent of the nonionic type is chosen from optionally oxyethylenated esters of sorbitol and of $C_8$-$C_{22}$ fatty acids and fromalkyl polyglucosides.

**5.** Composition according to any one of the preceding claims, characterized in that it additionally comprises at least

one anionic and/or amphoteric surface-active agent.

**6.** Composition according to any one of the preceding claims, characterized in that the silicone is of the volatile type having a boiling point of between 60°C and 260°C.

**7.** Composition according to any one of Claims 1 to 5, characterized in that the silicone is of the nonvolatile type and is chosen from polyalkylsiloxanes, polyarylsiloxanes, polyalkylarylsiloxanes, silicone gums and resins and organomodified polysiloxanes, and their mixtures.

**8.** Composition according to any one of Claims 1 to 5, characterized in that the hydrocarbon is a linear or branched, cyclic or acyclic $C_8$-$C_{300}$ hydrocarbon.

**9.** Composition according to Claim 8, characterized in that the hydrocarbon is chosen from isododecane, isohexadecane and its isomers, isoicosane, isotetracosane and their isomers.

**10.** Composition according to any one of Claims 1 to 5, characterized in that the fatty alcohol is a saturated or unsaturated, linear or branched $C_8$-$C_{22}$ fatty alcohol.

**11.** Composition according to Claim 10, characterized in that the fatty alcohol is chosen from 2-butyloctanol, lauryl alcohol, oleyl alcohol, isocetyl alcohol and isostearyl alcohol.

**12.** Composition according to any one of Claims 1 to 5, characterized in that the fatty ester is an ester of a $C_8$-$C_{22}$ fatty acid and of a $C_1$-$C_{22}$ alcohol or an ester of a $C_1$-$C_7$ acid or diacid and of a $C_8$-$C_{22}$ fatty alcohol.

**13.** Composition according to Claim 12, characterized in that the fatty ester is chosen from ethyl, isopropyl, 2-ethylhexyl and 2-octyldecyl palmitate, isopropyl, butyl, cetyl and 2-octyldecyl myristate, butyl and hexyl stearate, hexyl and 2-hexyldecyl laurate, isononyl isononanoate and dioctyl malate.

**14.** Composition according to any one of the preceding claims, characterized in that it additionally comprises at least one adjuvant chosen from fragrances, preservatives, sunscreen agents, sequestering agents, moisturizers or emollients, reducing agents, oxidizing agents, non-oily agents for conditioning the hair or the skin, colorants and acidifying or basifying agents.